# EUROPÄISCHE PATENTSCHRIFT

(11) **EP 1 598 358 B1**
(45) Veröffentlichungstag und Bekanntmachung des Hinweises auf die Patenterteilung: **03.12.2008**
(21) Anmeldenummer: 05009630.4
(22) Anmeldetag: 03.05.2005
(51) Int. Cl.: C07D 495/04, C08G 61/12, H01B 1/12

(54) **Verbindungen enthaltend 3,4-Methylendioxythiopen-Einheiten**
compounds comprising 3,4-methylendioxythiophene units
composes comprenant des unites 3,4-methylendioxythiophene

(30) Priorität: 15.05.2004 DE 102004024271
(43) Veröffentlichungstag der Anmeldung: 23.11.2005
(73) Patentinhaber: H.C. Starck GmbH, 38642 Goslar (DE)
(72) Erfinder: Brassat, Lutz, Dr., 51373 Leverkusen (DE); Kirchmeyer, Stephan, Dr., 51375 Leverkusen (DE); Reuter, Knud, Dr., 47800 Krefeld (DE)
(74) Vertreter: Clauswitz, Kai-Uwe Wolfram

(56) Entgegenhaltungen:
- AHONEN HJ ET AL: "Electrochemical synthesis and spectroscopic study of poly(3,4-methylenedioxythiophene)" SYNTHETIC METALS, Bd. 84, 1997, Seiten 215-216, XP002341697

## Beschreibung

Die Erfindung betrifft Verbindungen enthaltend gegebenenfalls substituierte 3,4-Methylendioxythiophen-Einheiten (Thieno[3,4-d]-1,3-dioxol-Einheiten), deren Herstellung sowie Verwendung als organische Halbleiter.

Das Feld molekularer Elektronik hat sich in den letzten 15 Jahren mit der Entdeckung organischer leitender und halbleitender Verbindungen rapide entwickelt. In dieser Zeit wurde eine Vielzahl von Verbindungen gefunden, die halbleitende oder elektrooptische Eigenschaften aufweisen. Es ist allgemeines Verständnis, dass die molekulare Elektronik nicht konventionelle Halbleiterbausteine auf der Basis von Silizium verdrängen wird. Stattdessen geht man davon aus, dass molekulare elektronische Bauelemente sich neue Anwendungsgebiete eröffnen werden, in denen die Eignung zur Beschichtung großer Flächen, strukturelle Flexibilität, Prozessierbarkeit bei niedrigen Temperaturen und niedrigen Kosten benötigt werden. Halbleitende organische Verbindungen werden derzeit für Anwendungsgebiete wie organische Feld-Effekt-Transistoren (OFET's), organische Lumineszenzdioden (OLED's), Sensoren und photovoltaische Elemente entwickelt. Durch einfache Strukturierung und Integration von OFET's in integrierte organische Halbleiterschaltungen werden preiswerte Lösungen für intelligente Karten (smart cards) oder Preisschilder möglich, die sich bislang mit Hilfe der Silizium-Technologie aufgrund des Preises und der mangelnden Flexibilität der Siliziumbausteine nicht realisieren lassen. Ebenfalls könnten OFET's als Schaltelemente in großflächigen flexiblen Matrixanzeigen verwendet werden. Eine Übersicht über organische Halbleiter, integrierte Halbleiterschaltungen und deren Anwendungen ist beispielsweise in Electronics 2002, Band 15, S. 38 beschrieben.

Bekannte halbleitende organische Verbindungen sind beispielsweise Polyfluorene und Fluorencopolymere, wie z.B. Poly(9,9-dioctylfluoren-co-bithiophen), mit dem Ladungsträgermobilitäten, im Folgenden auch kurz als Mobilitäten bezeichnet, bis 0,02 cm²/Vs erreicht wurden (Science, 2000, Band 290, S. 2123). Mit regioregulärem Poly(3-hexylthiophen-2,5-diyl) wurden sogar Mobilitäten bis zu 0,1 cm²/Vs erreicht (Science, 1998, Band 280, S. 1741). Weitere Vertreter halbleitender organischer Verbindungen sind beispielsweise Oligothiophene, insbesondere solche mit endständigen Alkylsubstituenten, und Pentacen. Typische Mobilitäten, für z.B. α,α'-Dihexyl-quater-, -quinque- und -sexithiophen liegen bei 0.05 - 0.1 cm²/Vs. Die vorangehend beschriebenen Verbindungen sind allerdings nur eingeschränkt für den Einsatz in (opto)elektronischen Bauteilen geeignet. So haben einige dieser Verbindungen beispielsweise Phasenumwandlungen, die ihren Einsatz oberhalb einer für die jeweilige Verbindung typischen Temperatur ausschließen, oder ihre Mobilitäten sind für manche Anwendungen nicht ausreichend.

Es hat mehrere Versuche gegeben, Oligomere aus Alkylendioxythiophen-Einheiten, insbesondere aus 3,4-Ethylendioxythiophen-Einheiten, herzustellen und als organische Halbleiter einzusetzen. Von Nachteil ist allerdings, dass diese Oligoalkylendioxythiophene, vor allem die entsprechenden 3,4-Ethylendioxythiophenverbindungen, sehr oxidationsempfindlich sind. Dadurch ist ihr Einsatz als organischer Halbleiter nur eingeschränkt möglich, da eine eventuelle Dotierung des organischen Halbleiters zu einer schlechten Strommodulation führen würde. Erst in jüngster Vergangenheit wurden von Roncali et al., Journal of Organic Chemistry, 2003, 68, 5357 - 5360 Synthesen von Oligo(3,4-ethylendioxythiophenen) beschrieben, die eine reduzierte Oxidationsempfindlichkeit aufweisen. Ergebnisse für diese Verbindung in Hinblick auf die Eignung für den Einsatz als organische Halbleiter in Transistoren oder anderen (opto)elektronischen Bauteilen sind allerdings bisher nicht bekannt.

Es besteht also weiterhin Bedarf an Verbindungen, die als organische Halbleiter verwendet werden können.

Aufgabe war es daher, neue halbleitende organische Verbindungen herzustellen, welche eine geringe Oxidationsempfindlichkeit aufweisen und gut für den Einsatz als organische Halbleiter in (opto)elektronischen Bauteilen geeignet sind.

Überraschend wurde nun gefunden, dass neutrale, d.h. in der nicht-oxidierten Form vorliegende, Verbindungen, die 3,4-Methylendioxythiophen-Einheiten, im Folgenden auch vereinfacht aber gleichbedeutend als Methylendioxythiophen-Einheiten bezeichnet, aufweisen, in hohem Maß oxidationsstabil sind und als Halbleiter verwendet werden können.

Gegenstand der vorliegenden Erfindung sind neutrale Verbindungen der allgemeinen Formel (I) 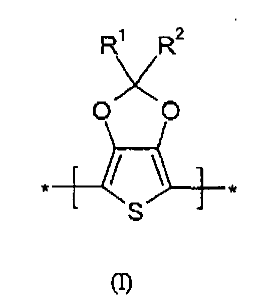 worin
- R¹ und R²: H darstellen
die Anzahl der wiederkehrenden Einheiten der allgemeinen Formel (I) n ist, wobei
- n: für eine ganze Zahl von 2 bis 1000, bevorzugt von 2 bis 200 steht
und die Verbindung Endgruppen R³ und R⁴ trägt, wobei
- R³ und R⁴: unabhängig voneinander für H oder eine lineare oder verzweigte C₁-C₂₀-Alkylgruppe, stehen.

In der Formel I kennzeichnet der Stern (*) jeweils eine Bindungsstelle für benachbarte Gruppen oder endständige Gruppen R³ oder R⁴.

Bei den erfindungsgemäßen Verbindungen handelt es sich um Polymere. Unter Polymeren sind im Rahmen der Erfindung alle Verbindungen umfasst, bei denen n eine ganze Zahl größer 1 ist. Des Weiteren sind unter Polymeren alle solchen Verbindungen zu verstehen, die entweder polydispers sind, d.h. eine Molgewichtsverteilung aufweisen, oder monodispers sind, d.h. ein einheitliches Molekulargewicht aufweisen. Bevorzugt sind die erfindungsgemäßen Verbindungen im Sinne der vorangehenden Definition monodispers.

R¹ und R² stehen in der allgemeinen Formel (I) für H,

R³ und R⁴ stehen in der allgemeinen Formel (I) unabhängig voneinander für H oder eine lineare oder verzweigte C₁-C₂₀-Alkylgruppe, wie z.B. Methyl-, Ethyl-, n-Propyl-, iso-Propyl-, n-Butyl-, sec-Butyl-, iso-Butyl-, tert-Butyl-, n-Pentyl-, 1-Methylbutyl-, 2-Methylbutyl-, 3-Methylbutyl-, 1-Ethylpropyl-, 1,1-Dimethylpropyl-, 1,2-Dimethylpropyl-, 2,2-Dimethylpropyl-, n-Hexyl-, n- Heptyl-, n-Octyl-, 2-Ethylhexyl-, n-Nonyl-, n-Decyl-, n-Undecyl-, n-Dodecyl-, n-Tridecyl-, n-Tetradecyl-, n-Hexadecyl-, n-Octadecyl- oder n-Eicosyl-,

Beispielhaft für die erfindungsgemäßen Verbindungen seien die Folgenden genannt:
Bis(methylendioxythiophen), Ter(methylendioxythiophen), Quater(methylendioxythiophen), Quinque(methylendioxythiophen), Sexi(methylendioxythiophen), Septi(methylendioxythiophen), Octi(methylendioxythiophen), Poly(methylendioxythiophen). Die Aufzählung dient der beispielhaften Erläuterung der Erfindung und ist nicht als abschließend zu verstehen.

Weiterhin bevorzugt Gegenstand der vorliegenden Erfindung sind solche erfindungsgemäßen Verbindungen, in denen R¹ und R² für H stehen.

Beispielhaft für solche erfindungsgemäßen Verbindungen seien die Folgenden aufgeführt:
2-Ethyl-bis(methylendioxythiophen), 2-Ethyl-ter(methylendioxythiophen), 2-Ethyl-quater-(methylendioxythiophen), 2-Ethyl-quinque(methylendioxythiophen), 2-Ethyl-sexi(methylendioxythiophen), 2-Ethyl-septi(methylendioxythiophen), 2-Ethyl-octi(methylendioxythiophen), 2-Propyl-bis(methylendioxythiophen), 2-Propyl-ter(methylendioxythiophen), 2-Propyl-quater-(methylendioxythiophen), 2-Propyl-quinque(methylendioxythiophen), 2-Propyl-sexi(methylendioxythiophen), 2-Propyl-septi(methylendioxythiophen), 2-Propyl-octi(methylendioxythiophen), 2-Butyl-bis(methylendioxythiophen), 2-Butyl-ter(methylendioxythiophen), 2-Butyl-quater(methylendioxythiophen), 2-Butyl-quinque(methylendioxythiophen), 2-Butyl-sexi(methylendioxythiophen), 2-Butyl-septi(methylendioxythiophen), 2-Butyl-octi(methylendioxythiophen), 2-Pentyl-bis-(methylendioxythiophen), 2-Pentyl-ter(methylendioxythiophen), 2-Pentyl-quater(methylendioxythiophen), 2-Pentyl-quinque(methylendioxythiophen), 2-Pentyl-sexi(methylendioxythiophen), 2-Pentyl-septi(methylendioxythiophen), 2-Pentyl-octi(methylendioxythiophen),2-Hexyl-bis-(methylendioxythiophen), 2-Hexyl-ter(methylendioxythiophen), 2-Hexyl-quater(methylendioxythiophen), 2-Hexyl-quinque(methylendioxythiophen), 2- Hexyl-sexi(methylendioxythiophen), 2-Hexyl-septi(methylendioxythiophen), 2-Hexyl-octi(methylendioxythiophen), 2,5'-Diethyl bis-(methylendioxythiophen), 2,5'-Dipropyl-bis(methylendioxythiophen), 2,5'-Dibutyl-bis(methylendioxythiophen), 2,5'-Dipentyl-bis(methylendioxythiophen), 2,5'-Dihexyl-bis(methylendioxythiophen), 2,5'-Dioctyl-bis(methylendioxythiophen), 2,5 " -Dimethyl-ter(methylendioxythiophen), 2,5 " -Diethyl-ter(methylendioxythiophen), 2,5''-Dipropyl-ter(methylendioxythiophen), 2,5''-Dibutyl-ter(methylendioxythiophen), 2,5"-Dihexylter(methylendioxythiophen), 2,5 ''-Dioctyl-ter(methylendioxythiophen), 2,5''-Didecyl-ter-(methylendioxythiophen), 2,5''-Didodecyl-ter(methylendioxythiophen), 2,5'''-Dimethyl-quater-(methylendioxythiophen), 2,5'''-Diethyl-quater(methylendioxythiophen), 2,5'''-Dihexyl-quater-(methylendioxythiophen), 2,5'''-Didecyl-quater(methylendioxythlophen), 2,5""-Diethyl-quinque-(methylendioxythiophen), 2,5''''-Dihexyl-quinque(methylendioxythlophen), 2,5''''-Didecylquinque(methylendioxythiophen), 2,5'''''-Dimethyl-sexi(methylendioxythiophen), 2,5'''''-Diethyl-sexi(methylendioxythiophen), Die Aufzählung dient der beispielhaften Erläuterung der Erfindung und ist nicht als abschließend zu verstehen.

Weiterhin bevorzugt Gegenstand der vorliegenden Erfindung sind solche erfindungsgemäßen Verbindungen, in denen R³ und R⁴ für H stehen.

In weiteren bevorzugten Ausführungsformen sind die erfindungsgemäßen Verbindungen solche der allgemeinen Formel (II), 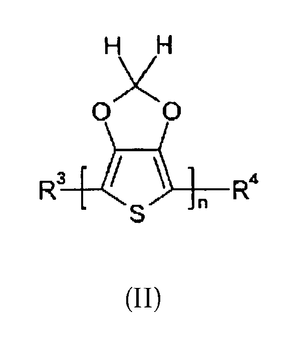 worin R¹ und R² für H stehen, und R³, R⁴ und n die vorangehend für die allgemeine Formel (I) aufgeführte Bedeutung haben. Vorzugsbereiche und Kombinationen dieser Vorzugsbereiche gelten analog.

In weiteren bevorzugten Ausführungsformen sind die erfindungsgemäßen Verbindungen solche der allgemeinen Formel (II-a), 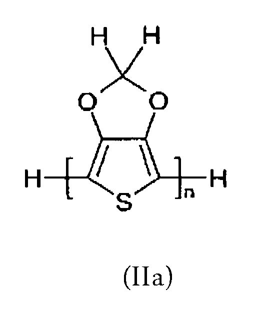 worin R¹, R², R³ und R⁴ für H stehen und n die vorangehend für die allgemeine Formel (I) aufgeführte Bedeutung hat. Vorzugsbereiche gelten analog.

Beispielhaft für solche erfindungsgemäßen Verbindungen der allgemeinen Formel (II-a) seien die Folgenden genannt:
Bis(methylendioxythiophen), Ter(methylendioxythiophen), Quater(methylendioxythiophen), Quinque(methylendioxythiophen), Sexi(methylendioxythiophen), Septi(methylendioxythiophen), Octi(methylendioxythiophen), Novi(methylendioxythiophen), Deci(methylendioxythiophen), Undeci(methylendioxythiophen), Dodeci(methylendioxythiophen) und Poly(methylendioxythiophen). Die Aufzählung dient der beispielhaften Erläuterung der Erfindung und ist nicht als abschließend zu verstehen.

Prinzipiell ist es möglich, die erfindungsgemäßen Verbindungen mittels unterschiedlicher, dem Fachmann grundsätzlich bekannter Verfahren basierend auf wenigstens einer metallorganischen Reaktion herzustellen.

Weiterhin Gegenstand der Erfindung ist daher ein Verfahren zur Herstellung einer erfindungsgemäßen Verbindung, wobei die erfindungsgemäße Verbindung durch wenigstens eine metallorganische Reaktion hergestellt wird.

Bevorzugt ist dies ein Verfahren, wobei die erfindungsgemäße Verbindung durch eine Kumada-Kupplung, Suzuki-Kupplung oder Stille-Kupplung hergestellt wird.

In einer bevorzugten Ausführungsform werden die erfindungsgemäßen Verbindungen mittels einer Variante der Suzuki-Kupplung, häufig auch als Suzuki-Kondensation bezeichnet, hergestellt. Die Suzuki-Kondensation bzw. Suzuki-Kupplung, d.h. die Umsetzung von Arylhalogeniden und Arylboronsäure-Verbindungen mit einer Pd-Verbindung als Katalysator in Gegenwart einer Base, ist z.B. in Suzuki et al., Chem. Rev. 1995, 95, 2457-2483 beschrieben. In einer bevorzugten Ausführungsform wird das erfindungsgemäße Verfahren gemäß einer erfindungsgemäßen Variante dieser Suzuki-Kupplung durchgeführt, wobei Organylhalogenide bzw. Organyl-Boronsäureester gegebenenfalls in Anwesenheit wenigstens einer Base und/oder wenigstens eines Katalysators, der ein Metall der VIII. Nebengruppe des Periodensystems der Elemente, im Folgenden kurz als Metall der VIII. Nebengruppe bezeichnet, enthält, umgesetzt werden.

Die bevorzugte Ausführungsform des erfindungsgemäßen Verfahrens (Suzuki-Kupplung) wird bei einer Temperatur von +20°C bis +200°C, bevorzugt von +40°C bis +150°C, besonderes bevorzugt von +80°C bis +130°C, in einem organischen Lösungsmittel oder Lösungsmittelgemisch durchgeführt.

Als Katalysatoren, die ein Metall der VIII. Nebengruppe enthalten, kommen prinzipiell alle geeigneten Verbindungen in Frage, die ein Metall der VIII. Nebengruppe, bevorzugt Pd, Ni oder Pt, besonders bevorzugt Pd, enthalten. Der oder die Katalysator(en) werden bevorzugt in Mengen von 0,05 Gew.-% bis 10 Gew.-%, besonders bevorzugt von 0,5 Gew.-% bis 5 Gew.-%, bezogen auf das Gesamtgewicht der zu kuppelnden Verbindungen eingesetzt.

Besonders geeignete Katalysatoren sind Komplexverbindungen von Metallen der VIII. Nebengruppe, insbesondere Komplexe des Palladium(0), die an Luft stabil sind, Pd-Komplexe, die sich leicht mit Organometallreagenzien (z.B. Lithiumalkylverbindungen oder magnesiumorganischen Verbindungen) oder Phosphinen zu Palladium(0)-Komplexen reduzieren lassen, oder Palladium(2)-Komplexe gegebenenfalls unter Zusatz von PPh₃ oder anderen Phosphinen. Beispielsweise können PdCl₂(PPh₃)₂, PdBr₂(PPh₃)₂ oder Pd(OAc)₂ oder Mischungen aus diesen Verbindungen unter Zusatz von PPh₃ eingesetzt werden. Bevorzugt wird Pd(PPh₃)₄ ohne oder unter Zusatz von Phosphinen, in einer bevorzugten Ausführungsform ohne Zusatz von Phosphinen, eingesetzt, welches in preiswerter Form zur Verfügung steht. Als Phosphine werden bevorzugt PPh₃, PEtPh₂, PMePh₂, PEt₂Ph oder PEt₃, besonders bevorzugt PPh₃, eingesetzt.

Es ist jedoch auch möglich, als Katalysatoren Palladiumverbindungen ohne Phosphinzusatz einzusetzen, wie beispielweise Pd(OAc)₂.

Als Base können beispielweise Hydroxide, wie z.B. NaOH, KOH, LiOH, Ba(OH)₂, Ca(OH)₂, Alkoxide, wie z.B. NaOEt, KOEt, LiOEt, NaOMe, KOMe, LiOMe, Alkalimetallsalze von Carbonsäuren, wie z.B. Natrium-, Kalium- oder Lithiumcarbonat, -hydrogencarbonat, -acetat, -citrat, -acetylacetonat, -glycinat, oder andere Carbonate, wie z.B. Cs₂CO₃ oder Tl₂CO₃, Phosphate, wie z.B. Natriumphosphat, Kaliumphosphat oder Lithiumphosphat, oder Mischungen aus diesen eingesetzt werden. Bevorzugt wird Natriumcarbonat eingesetzt. Die Basen können als Lösungen in Wasser oder als Suspensionen in organischen Lösungsmitteln, wie Toluol, Dioxan oder DMF, eingesetzt werden. Bevorzugt sind Lösungen in Wasser, da die erhaltenen Produkte aufgrund ihrer geringen Löslichkeit in Wasser so vom Reaktionsgemisch leicht abgetrennt werden können.

Es ist auch möglich weitere Salze, wie beispielweise LiCl oder LiBr, als Hilfsmittel einzusetzen.

Als organische Lösungsmittel kommen grundsätzlich alle Lösungsmittel oder Lösungsmittelgemische in Frage, welche mit den Boronsäureestern nicht reagieren. Dies sind in der Regel Verbindungen, welche keine Halogenatome oder keine gegenüber Boronsäureestern reaktiven Wasserstoffatome aufweisen. Geeignete Lösungsmittel sind beispielsweise Alkane wie Pentan, Hexan und Heptan, Aromaten wie Benzol, Toluol und Xylole, Ethergruppen enthaltende Verbindungen wie Dioxan, Dimethoxyethan und Tetrahydrofuran und polare Lösungsmittel wie Dimethylformamid oder Dimethylsulfoxid. Bevorzugt werden im erfindungsgemäßen Verfahren Aromaten als Lösungsmittel eingesetzt. Ganz besonders bevorzugt ist Toluol. Es ist auch möglich, als Lösungsmittel Mischungen aus zwei oder mehreren dieser Lösungsmittel einzusetzen.

Die in diesem Verfahren eingesetzten Organylhalogenide können nach bekannten Verfahren hergestellt werden oder sind käuflich zu erwerben. Die Herstellung der Boronsäureester kann beispielsweise durch die Umsetzung von Arylhalogeniden und Bis(organyl)diboran durch metallkatalysierte Kupplung (WO-A 01/29051 A1, Tetrahedron Lett. 2002, S. 5649), durch Kupplung von Oligothiophenhalogeniden mit beispielsweise Pinakolboran erfolgen (J. Org. Chem. 1997, B.62, S. 6458; J. Organomet. Chem. 2001, B. 640, S. 197; Chem. Commun. 2002, S. 1566) oder durch Umsetzung von metallorganischen Verbindungen, z.B. magnesiumorganischen Verbindungen (z.B. Grignard-Verbindungen) oder lithiumorganischen Verbindungen, mit Boronsäureestern erfolgen. Diese Verfahren sind dem Fachmann bekannt.

In einer weiteren bevorzugten Ausführungsform werden die erfindungsgemäßen Verbindungen mittels einer Kumada-Kupplung hergestellt. Die Kumada-Kupplung, d.h. die Umsetzung eines Arylhalogeniden und einer Arylgrignard-Verbindung in Gegenwart eines Pd- oder eines Ni-Katalysators ist z.B. in Kumada et al., J. Am. Chem. Soc. 1972, 94, 4373 - 4376 beschrieben. In einer bevorzugten Ausführungsform wird das erfindungsgemäße Verfahren gemäß einer erfindungsgemäßen Variante dieser Kumada-Kupplung durchgeführt, wobei Aryl- bzw. Heteroarylhalogenide und Grignardverbindungen von Aryl- bzw. Heteroarylhalogeniden in Anwesenheit eines Katalysators, der ein Metall der VIII. Nebengruppe des Periodensystems der Elemente, im Folgenden kurz als Metall der VIII. Nebengruppe bezeichnet, enthält, umgesetzt werden. Die bevorzugte Ausführungsform des erfindungsgemäßen Verfahrens (Kumada-Kupplung) wird bei einer Temperatur von 0°C bis 200 °C, bevorzugt von + 20 °C bis + 150 °C, besonders bevorzugt von + 40 °C bis + 130 °C, in einem organischen Lösungsmittel oder einem Lösungsmittelgemisch durchgeführt

Als Katalysatoren, die ein Metall der VIII. Nebengruppe enthalten, kommen prinzipiell alle geeigneten Verbindungen in Frage, die ein Metall der VIII. Nebengruppe, bevorzugt Pd oder Ni, besonders bevorzugt Pd, enthalten. Der oder die Katalysator(en) werden bevorzugt in Mengen von 0,05 Gew.-% bis 10 Gew.-%, besonders bevorzugt von 0,5 Gew.-% bis 5 Gew.-%, bezogen auf das Gesamtgewicht der zu kuppelnden Verbindungen eingesetzt.

Besonders geeignete Katalysatoren sind Komplexverbindungen von Metallen der VIII. Nebengruppe, insbesondere Komplexe des Palladium(0), die an Luft stabil sind, Pd-Komplexe, die sich leicht mit Organometallreagenzien (z.B. Lithiumalkylverbindungen oder magnesiumorganischen Verbindungen) oder Phosphinen zu Palladium(0)-Komplexen reduzieren lassen, oder Palladium(2)-Komplexe gegebenenfalls unter Zusatz von PPh₃ oder anderen Phosphinen. Beispielsweise können PdCl₂(PPh₃)₂, PdBr₂(PPh₃)₂ oder Pd(OAc)₂ oder Mischungen aus diesen Verbindungen unter Zusatz von Diphenylphosphinoethan (dppe) oder Diphenylphosphinopropan (dppp) oder 1,1'-Bis(diphenylphoshino)ferrocen (dppf) eingesetzt werden. Bevorzugt werden PdCl₂(dppe), PdCl₂(dppp) und PdCl₂(dppf) als Katalysatoren eingesetzt.

Als organische Lösungsmittel kommen grundsätzlich alle Lösungsmittel oder Lösungsmittelgemische in Frage, welche mit den Grignardreagentien nicht reagieren. Dies sind in der Regel Verbindungen, welche keine Halogenatome oder keine gegenüber Grignardverbindungen reaktiven Wasserstoffatome aufweisen. Geeignete Lösungsmittel sind beispielsweise Aromaten wie Benzol, Toluol und Xylole, Ethergruppen enthaltende Verbindungen wie Dioxan, Dimethoxyethan, Diethylether, Dibutylether und Tetrahydrofuran. Bevorzugt werden im erfindungsgemäßen Verfahren etherische Lösungsmittel eingesetzt. Ganz besonders bevorzugt ist Tetrahydrofuran. Es ist auch möglich, als Lösungsmittel Mischungen aus zwei oder mehreren dieser Lösungsmittel einzusetzen.

In einer weiteren bevorzugten Ausführungsform werden die erfindungsgemäßen Verbindungen mittels einer Stille-Kupplung hergestellt. Die Stille-Kupplung, d.h. die Umsetzung eines Arylhalogeniden und einer Aryl- oder Alkenylstannyl-Verbindung in Gegenwart eines Pd -Katalysators ist z.B. in Stille et al., Angew. Chem. 1986, 98, 504 beschrieben. In einer bevorzugten Ausführungsform wird das erfindungsgemäße Verfahren gemäß einer erfindungsgemäßen Variante dieser Stille-Kupplung durchgeführt, wobei Aryl- bzw. Heteroarylhalogenide und Aryl- und Alkenyl-Stannylverbindungen in Anwesenheit eines Katalysators, der ein Metall der VIII. Nebengruppe des Periodensystems der Elemente, im Folgenden kurz als Metall der VIII. Nebengruppe bezeichnet, enthält, umgesetzt werden. Die bevorzugte Ausführungsform des erfindungsgemäßen Verfahrens (Stille-Kupplung) wird bei einer Temperatur von 0°C bis 200 °C, bevorzugt von + 20 °C bis + 150 °C, besonders bevorzugt von + 40 °C bis + 130 °C, in einem organischen Lösungsmittel oder einem Lösungsmittelgemisch durchgeführt

Als Katalysatoren, die ein Metall der VIII. Nebengruppe enthalten, kommen prinzipiell alle geeigneten Verbindungen in Frage, die ein Metall der VIII. Nebengruppe, besonders bevorzugt Pd, enthalten. Der oder die Katalysator(en) werden bevorzugt in Mengen von 0,05 Gew.-% bis 10 Gew.-%, besonders bevorzugt von 0,5 Gew.-% bis 5 Gew.-%, bezogen auf das Gesamtgewicht der zu kuppelnden Verbindungen eingesetzt.

Besonders geeignete Katalysatoren sind Komplexverbindungen von Metallen der VIII. Nebengruppe, insbesondere Komplexe des Palladium(0), die an Luft stabil sind, Pd-Komplexe, die sich leicht mit Organometallreagenzien (z.B. Lithiumalkylverbindungen oder magnesiumorganischen Verbindungen) oder Phosphinen zu Palladium(0)-Komplexen reduzieren lassen, oder Palladium(2)-Komplexe gegebenenfalls unter Zusatz von PPh₃ oder anderen Phosphinen. Beispielsweise können PdCl₂(PPh₃)₂, PdBr₂(PPh₃)₂ oder Pd(OAc)₂ oder Mischungen aus diesen Verbindungen unter Zusatz von PPh₃ eingesetzt werden. Bevorzugt wird Pd(PPh₃)₄ ohne oder unter Zusatz von Phosphinen, in einer bevorzugten Ausführungsform ohne Zusatz von Phosphinen, eingesetzt, welches in preiswerter Form zur Verfügung steht. Als Phosphine werden bevorzugt PPh₃, PEtPh₂, PMePh₂, PEt₂Ph oder PEt₃, besonders bevorzugt PPh₃, eingesetzt.

Es ist jedoch auch möglich, als Katalysatoren Palladiumverbindungen ohne Phosphinzusatz einzusetzen, wie beispielweise Pd(OAc)₂.

Als organische Lösungsmittel kommen grundsätzlich alle Lösungsmittel oder Lösungsmittelgemische in Frage, welche mit den Stannylverbindungen nicht reagieren. Dies sind in der Regel Verbindungen, welche keine Halogenatome oder keine gegenüber Stannylverbindungen reaktiven Wasserstoffatome aufweisen. Geeignete Lösungsmittel sind beispielsweise Aromaten wie Benzol, Toluol und Xylole, Ethergruppen enthaltende Verbindungen wie Dioxan, Dimethoxyethan, Diethylether, Dibutylether und Tetrahydrofuran, oder polare Lösungsmittel wie Dimethylformamid, N-Methylpyrrolidon oder Acetonitril. Es ist auch möglich, als Lösungsmittel Mischungen aus zwei oder mehreren dieser Lösungsmittel einzusetzen.

Die Aufarbeitung der jeweiligen Reaktionsmischung erfolgt nach an sich bekannten Verfahren, z.B. durch Verdünnen, Fällen, Filtration, Extraktion, Waschen, Rekristallisation aus geeigneten Lösungsmitteln, Chromatographie und/oder Sublimation. Beispielsweise kann eine Aufarbeitung in der Weise erfolgen, dass die Reaktionsmischung nach vervollständigter Reaktion in ein Gemisch aus saurem (Eis-)Wasser, z.B. hergestellt aus 1 molarer Salzsäure, und Toluol gegossen, die organische Phase abgetrennt, mit Wasser gewaschen, das als Feststoff enthaltene Produkt abfiltriert, mit Toluol gewaschen und anschließend im Vakuum getrocknet wird. Die erfindungsgemäßen Verbindungen können bereits ohne weitere anschließende Reinigungsprozesse in hoher Qualität und Reinheit erhalten werden. Es ist jedoch möglich, diese Produkte nach bekannten Verfahren, z.B. durch Rekristallisation, Chromatographie oder Sublimation weiter zu reinigen.

Die erfindungsgemäßen Verbindungen sind elektrisch neutral und halbleitend und weisen eine geringe Oxidationsempfindlichkeit auf. Sie lassen sich zudem gut aus Lösung applizieren. Folglich eignen sie sich gut für den Einsatz als organische Halbleiter in (opto)elektronischen Bauteilen.

Dies ist insofern überraschend, als dass die monomere Stammverbindung 3,4-Methylendioxythiophen bzw. Thieno[3,4-d]-1,3-dioxol, dem Fachmann aus einer Reihe von Veröffentlichungen bekannt ist und er annehmen musste, dass sich Verbindungen mit Methylendioxythiophen-Einheiten analog zu anderen 3,4-Alkylendioxythiophen-Einheiten enthaltenden Verbindungen verhalten. Somit war zu erwarten, dass Methylendioxythiophen-Einheiten enthaltende Verbindungen einen stabilen elektrisch geladenen bzw. oxidierten Zustand aufweisen und der neutrale Zustand eher instabil ist. So werden z.B. von Ahonen et al., Synthetic Metals (1997), 84(1-3), 215-216 Polymere des Methylendioxythiophens nur in oxidierter, d.h. kationischer Form beschrieben, die somit nicht als Halbleiter, sondern als organische Leiter eingesetzt werden können (vgl. EP-A 339 340). Nicht-oxidierte, d.h. neutrale Verbindungen mit 3,4-Methylendioxythiophen-Einheiten sind bislang in der Literatur nicht beschrieben.

Weiterhin Gegenstand der vorliegenden Erfindung ist daher die Verwendung der erfindungsgemäßen Verbindungen als organische Halbleiter in elektronischen Bauelementen, in aktiven und lichtemittierenden, elektronischen Bauelementen wie Feldeffekt-Transistoren, organischen Lumineszenzdioden, photovoltaischen Zellen, Lasern oder Sensoren.

Dazu werden die erfindungsgemäßen Verbindungen in Form von Schichten auf geeignete Substrate, zum Beispiel auf mit elektrischen oder elektronischen Strukturen versehene Silizium-Wafer, Polymerfolien oder Glasscheiben aufgetragen. Für den Auftrag kommen prinzipiell sämtliche dem Fachmann bekannten Auftragsverfahren in Betracht. Beispielsweise können die Verbindungen der allgemeinen Formel (I) aus der Gasphase oder aus Lösung aufgetragen werden, wobei man das Lösungsmittel anschließend verdampft. Das Aufbringen aus Lösung kann nach den bekannten Verfahren, beispielsweise durch Sprühen, Tauchen, Drucken und Rakeln, Spin-Coating und durch Ink-Jet-Druck erfolgen. Die erfindungsgemäßen Verbindungen können auch aus der Gasphase, z.B. durch Aufdampfen, aufgebracht werden. Auf diese Weise können Schichten mit den geringsten Defekten und höchsten Ladungsmobilitäten erhalten werden.

Weiterhin Gegenstand der vorliegenden Erfindung ist daher ein elektronisches Bauelement enthaltend wenigstens eine erfindungsgemäße Verbindung.

Die folgenden Beispiele dienen der beispielhaften Erläuterung und Veranschaulichung der Erfindung, stellen jedoch keine Beschränkung dar.

### Beispiele

### Beispiel 1: Synthese von Bis(methylendioxythiopen) (II-a-1) (Bis-MDT)

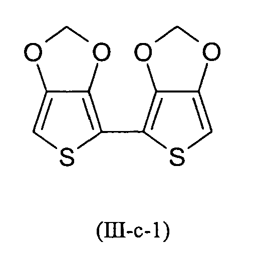

3,96 g 3,4-Methylendioxythiophen werden unter N₂-Atmosphäre in 100 ml absolutiertem (abs.) Tetrahydrofuran (THF) gelöst und auf 0°C gekühlt. Zu der auf 0°C gekühlten Lösung werden 20 ml 1,6-M n-Butyllithium-Lösung in n-Hexan getropft. Die Mischung wird 30 min bei 0°C gerührt. Danach werden 4,41 g CuCl₂ auf einmal zugegeben und die Mischung anschließend 12 h bei 23°C gerührt. Nach Eingießen in Eis/Wasser werden 1,9 g (= 48 % der Theorie) Bis(3,4-methylendioxythiophen) (II-a-1) abgesaugt.
Schmp.: 225 - 231°C

| | | | | |
|---|---|---|---|---|
| Elementaranalyse: | Gemessen: | C: 46,7 % | H: 2,25 % | S: 24,6 % |
| | Berrechnet: | C: 47,0 %) | H: 2,37 % | S: 25,6 % (für C₁₀H₆O₄S₂) |

¹H-NMR-Spektrum (CDCl₃; ppm δ gegen TMS): 6,00 (2H), 6,28 (4H)

### Beispiel 2: Synthese von 2-Hexyl-bis(methylendioxythiophen) (II-1)

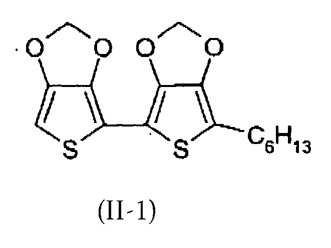

In 20 ml wasserfreiem THF werden bei -20°C 3,52 ml 2,5-M n-Butyllithium-Lösung in Hexan zugegeben. Die Mischung wird 1 h gerührt und anschließend 2,03 g Bis-MDT (II-1), hergestellt gemäß Beispiel 1, in 50 ml THF zugegeben. Die Mischung wird eine weitere Stunde bei -20°C gerührt und anschließend werden bei -20°C 1,65 g Hexylbromid zugegeben. Die Reaktionsmischung wird aufgetaut und mit Wasser hydrolysiert. Die wässrige Phase wird dreimal mit je 50 mL Methylenchlorid extrahiert und das Lösungsmittel aus den vereinigten organischen Phasen vollständig entfernt. Es werden 0,7 g 2-Hexyl-bis(methylendioxythiophen) nach Chromatographie an Kieselgel als hellgrauer Feststoff erhalten .

### Beispiel 3: Synthese von 2,5'''-Dihexyl-quater(methylendioxythiophen) (II-2)

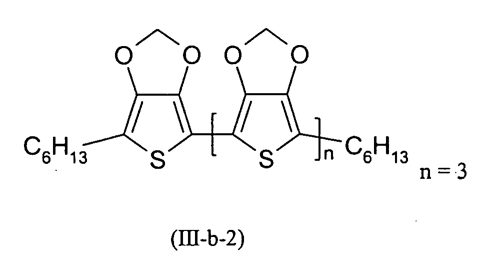

In 20 ml THF werden bei -70°C 1 ml 1,6-M n-Butyllithium-Lösung in n-Hexan vorgelegt. Anschließend werden 0,157 ml Diisopropylamin zugetropft und die Mischung 1 h gerührt. Anschließend werden bei -78°C 0,5 g 2-Hexyl-bis(methylendioxythiophen) - hergestellt gemäß Beispiel 2 - zugetropft. Die Mischung wird auf -20°C aufgetaut und 1 h gerührt. Hiernach wird erneut auf -78°C abgekühlt und ca. 0,16 g wasserfreies Kupfer(II)chlorid hinzugegeben. Die Mischung wird 1 h bei -70°C gerührt und anschließend auf 23°C aufgetaut. Anschließend wird mit Wasser hydrolysiert, die wässrige Phase dreimal mit je 50 ml mit Methylenchlorid extrahiert und das Lösungsmittel aus den vereinigten organischen Phasen vollständig entfernt. Es werden 0,24 g 2,5'''-Dihexyl-quater(methylendioxythiophen) (II-2) als gelb-braunes Pulver erhalten.

## Patentansprüche

1. Neutrale Verbindung der allgemeinen Formel (I) 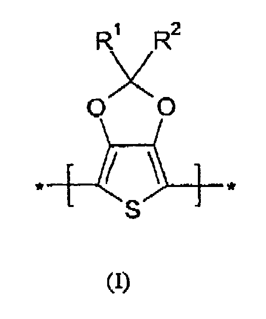 worin
R¹ und R² H, darstellen
die Anzahl der wiederkehrenden Einheiten der allgemeinen Formel (I) n ist, wobei
n für eine ganze Zahl von 2 bis 1000 steht
und die Verbindung Endgruppen R³ und R⁴ trägt, wobei
R³ und R⁴ unabhängig voneinander für H, oder eine lineare oder verzweigte C₁-C₂₀. Alkylgruppe, stehen.

2. Verbindung gemäß Anpruch 1, **dadurch gekennzeichnet, dass** R³ und R⁴ für H stehen.

3. Verfahren zur Herstellung einer Verbindung gemaß Anspruch 1 oder 2 **dadurch gekennzeichnet, dass** die Verbindung durch wenigstens eine metallorganische Reaktion hergestellt wird.

4. Verfahren gemäß Anspruch 3, **dadurch gekennzeichnet, dass** die Verbindung durch eine Kumada-Kupplung, Suzuki-Kupplung oder Stille-Kupplung hergestellt wird.

5. Verwendung der Verbindungen gemäß Anspruch 1 oder 2 als organischer Halbleiter in elektronischen Bauelementen.

6. Verwendung gemäß Anspruch 5, **dadurch gekennzeichnet, dass** die elektronischen Bauelemente aus der Gruppe: Feld-Effekt-Transistoren, lichtemittierende Bauelemente wie organische Lumineszenzdioden, photovoltaische Zellen, Laser und Sensoren ausgewählt sind.

7. Elektronisches Bauelement, **dadurch gekennzeichnet, dass** es wenigstens eine Verbindung gemäß Anspruch 1 oder 2 enthält.

## Claims

1. Neutral compound of the general formula (I) 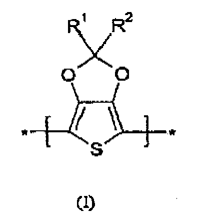 where
R¹ and R² denote H,
the number of repeating units of the general formula (I) is n, where
n denotes an integer from 2 to 1000,
and the compound has terminal groups R³ and R⁴, where
R³ and R⁴ independently of one another denote H or a linear or branched C₁-C₂₀ alkyl group.

2. Compound according to Claim 1, **characterized in that** R³ and R⁴ denote H.

3. Process for the production of a compound according to Claim 1 or 2, **characterized in that** the compound is produced by at least one organometallic reaction.

4. Process according to Claim 3, **characterized in that** the compound is produced by a Kumada coupling, Suzuki coupling or Stille coupling.

5. Use of the compounds according to Claim 1 or 2 as organic semiconductors in electronic components.

6. Use according to Claim 5, **characterized in that** the electronic components are selected from the group: field-effect transistors, light-emitting components such as organic light-emitting diodes, photovoltaic cells, lasers and sensors.

7. Electronic component, **characterized in that** it comprises at least one compound according to Claim 1 or 2.

## Revendications

1. Composé neutre de formule générale (I) 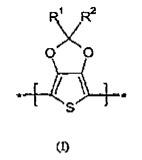 dans laquelle
R¹ et R² représentent un atome d'hydrogène,
le nombre de motifs récurrents de formule générale (I) est n, où
n représente un entier de 2 à 1000,
et le composé porte des groupes terminaux R³ et R¹, où
R³ et R⁴ représentent, indépendamment l'un de l'autre, un atome d'hydrogène ou un groupe alkyle en C₁-C₂₀ linéaire ou ramifié.

2. Composé selon la revendication 1, **caractérisé en ce que** R³ et R⁴ représentent un atome d'hydrogène.

3. Procédé de préparation d'un composé selon la revendication 1 ou 2, **caractérisé en ce que** le composé est préparé par au moins une réaction organométallique.

4. Procédé selon la revendication 3, **caractérisé en ce que** le composé est préparé par un couplage de Kumada, un couplage de Suzuki ou un couplage de SLille.

5. Utilisation des composés selon la revendication 1 ou 2 en tant que semi-conducteurs organiques dans des composants électroniques.

6. Utilisation selon la revendication 5, **caractérisée en ce que** les composants électroniques sont choisis parmi le groupe constitué de transistors à effet de champs, de composants photo-émetteurs tels que des diodes électroluminescentes organiques, des cellules photovoltaïques, des lasers et des capteurs.

7. Composant électronique, **caractérisé en ce qu'**il contient au moins un composé selon la revendication 1 ou 2.
